(19) Europäisches Patentamt
European Patent Office
Office européen des brevets

(11) **EP 2 505 229 A2**

(12) **EUROPÄISCHE PATENTANMELDUNG**

(43) Veröffentlichungstag:
**03.10.2012 Patentblatt 2012/40**

(51) Int Cl.:
*A61N 1/08* ^(2006.01)

(21) Anmeldenummer: **12159238.0**

(22) Anmeldetag: **13.03.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **30.03.2011 US 201161469107 P**

(71) Anmelder: **BIOTRONIK SE & Co. KG**
**12359 Berlin (DE)**

(72) Erfinder:
• **Bartels, Klaus**
**10115 Berlin (DE)**

• **Knorr, Stefan**
**10119 Berlin (DE)**
• **Frenzel, Timo**
**12435 Berlin (DE)**
• **Kolberg, Gernot**
**12161 Berlin (DE)**
• **Maxfield, Michelle**
**10967 Berlin (DE)**

(74) Vertreter: **Lindner-Vogt, Karin L.**
**Biotronik GmbH & Co. KG**
**Corporate Intellectual Properties**
**Woermannkehre 1**
**12359 Berlin (DE)**

(54) **Implantierbares Gerät**

(57) Die Erfindung betrifft ein temporär in einen Körper einführbares oder dauerhaft in einen Körper implantierbares Gerät - beispielsweise eine Elektrodenleitung - mit wenigstens einem langgestreckten elektrischen Funktionsleiter für die Übertragung von Therapiesignalen oder Diagnosesignalen oder beidem, sowie mit einem mit dem Funktionsleiter verbundenen und an einem proximalen Ende des Funktionsleiters angeordneten Stecker zum Anschließen des Gerätes an ein weiteres Gerät. Zwischen dem Funktionsleiter und wenigstens einem weiteren Leiter herrscht ein Leitungswellenwiderstand. Erfindungsgemäß ist ein Übergangsbereich von dem Funktionsleiter zu dem Stecker derart ausgebildet, dass ein Leitungswellenwiderstand zwischen dem Funktionsleiter und dem weiteren Leiter in dem Übergangsbereich so bemessen ist, dass er zwischen dem entsprechenden Leitungswellenwiderstand des Gerätes in einem distal des Übergangsbereichs gelegenen Leitungsabschnitt und dem Leitungswellenwiderstand liegt, der bei an ein weiteres Gerät angeschlossenem Stecker proximal des Übergangsbereichs herrscht.

FIG. 1

EP 2 505 229 A2

**Beschreibung**

Die Erfindung betrifft ein permanent oder temporär implantierbares Gerät mit einem langgestreckten elektrischen Leiter.

Solche Geräte, beispielsweise Elektrodenleitungen für die Elektrostimulation und/oder zur Wahrnehmung von elektronischen Signalen, haben den Nachteil, dass sich ihr elektrischer Leiter in einem Kernspintomografen (auch als Magnetresonanztomografen bezeichnet) erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Diese Erwärmung kann das Körpergewebe nachhaltig schädigen und soll möglichst gering sein. Im Vergleich zu herkömmlichen Implantaten soll die Erwärmung verringert werden. Deshalb können Herzschrittmacherpatienten heutzutage in der Regel nicht oder nur eingeschränkt in einem Kernspintomografen untersucht werden.

An implantierbaren Herzschrittmachern oder Defibrillatoren (im Folgenden auch gemeinsam als Herzstimulatoren oder IPG (implantable pulse generator) bezeichnet) sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Der Erfindung liegt die Aufgabe zugrunde, ein Gerät zu schaffen, welches eine Erwärmung infolge von durch einen Kernspintomografen eingestreuter Energie und damit eine Erwärmung an den Kontaktflächen zum umliegenden Gewebe verringert.

Erfindungsgemäß wird diese Aufgabe durch ein temporär in einen Körper einführbares oder dauerhaft in einen Körper implantierbares Gerät mit wenigstens einem langgestreckten elektrischen Funktionsleiter für die Übertragung von Therapiesignalen oder Diagnosesignalen oder beidem, sowie mit einem mit dem Funktionsleiter verbundenen und an einem proximalen Ende des Funktionsleiters angeordneten Stecker zum Anschließen des Gerätes an ein weiteres Gerät, wobei zwischen dem Funktionsleiter und wenigstens einem weiteren Leiter ein Leitungswellenwiderstand herrscht, dadurch gekennzeichnet, dass ein Übergangsbereich von dem Funktionsleiter zu dem Stecker derart ausgebildet ist, dass ein Leitungswellenwiderstand zwischen dem Funktionsleiter und dem weiteren Leiter in dem Übergangsbereich so bemessen ist, dass er zwischen dem entsprechenden Leitungswellenwiderstand des Gerätes in einem distal des Übergangsbereichs gelegenen Leitungsabschnitt und dem Leitungswellenwiderstand liegt, der bei an ein weiteres Gerät angeschlossenem Stecker proximal des Übergangsbereichs herrscht. Der Terminus Leitungswellenwiderstand umfasst hier sowohl den Gleichstromwiderstand als auch die frequenzabhängige Impedanz.

Mit anderen Worten betrifft die Erfindung ein langgestrecktes Implantat, das Verwendung findet für zur Stimulation und Ableitung von Signalen an Nerven, Gehirn, Herz und anderen Organen oder für die Zuleitung von implantierbaren Sensoren, mit mindestens einer Zuleitung, die ein proximal angeordnetes Gerät mit einer distal angeordneten Elektrode verbindet, wobei im Steckerbereich eine Änderung der geometrischen und elektrischen Verhältnisse vorgenommen ist, ohne die therapeutische Funktion zu stören. Diese Veränderung bewirkt eine Veränderung des Leitungswellenwiderstands zwischen Innen- und Außenleiter für eingekoppelte elektromagnetische Wellen.

Der Leitungswellenwiderstand (engl. characteristic impedance) beschreibt bei längshomogenen Leitungen, z. B. Kabel oder Einzeldrahtanordnungen, die aus wenigstens zwei elektrischen Leitern bestehen, das Verhältnis sich in eine gemeinsame Richtung ausbreitender Strom- und Spannungswellen zueinander.

Das implantierbare medizinische Gerät ist vorzugsweise eine Elektrodenleitung zum Anschluss an ein implantierbares medizinisches Gerät, wobei die Elektrodenleitung an ihrem proximalen Ende den Stecker aufweist und an ihrem distalen Ende wenigstens einen Elektrodenpol, der über den wenigstens einen Funktionsleiter mit dem Stecker elektrisch verbunden ist.

Vorzugsweise ist im Übergangsbereich des Gerätes eine Füllung angeordnet, die den Leitungswellenwiderstand beeinflusst und deren Eigenschaften und Form so gewählt sind, dass sich der gewünschte Leitungswellenwiderstand im Übergangsbereich ergibt.

Der Übergangsbereich ist vorzugsweise koaxial aufgebaut.

Besonders bevorzugt ist es, wenn die Füllung ein Material enthält, das wenigstens in einem Arbeitsfrequenzbereich von Kernspintomografen dissipativ wirkt. Eine solche Füllung hat die Wirkung, dass von einem Kernspintomografen induzierte Wellen (und damit Ströme) gedämpft werden. Dabei kommt es zu einer Erwärmung des Übergangsbereichs und eine Gewebeerwärmung im Bereich eines distalen Endes des Gerätes bzw. eine Gewebeerwärmung der Elektrodenleitung wird vermindert. Wenn der Übergangsbereich dissipativ gestaltet ist bedeutet dies bezogen auf den Leitungswellenwiderstand, dass dessen Imaginärteil größer ist, als bei einem weniger dissipativen Übergangsbereich.

Vorzugsweise ist für eine Isolierung im Steckerbereich ein Material als Füllung gewählt, dass den Leitungswellenwiderstand sinnvoll, d.h. im Sinne eines gewünschten Leitungswellenwiderstands verändert. Aufgrund dieser Änderung kann der Reflexions- und Transmissionsfaktor einen Bereich zwischen $R_{S,W}[0;1]$ und $T_{S,W}[-1,1]$ annehmen.

Der Faktor ist dabei abhängig von der Differenz der Leitungswellenwiderstände zwischen Stecker $Z_S$ und Wendelbereich $Z_W$.

$$R_{S,W} = \frac{Z_S - Z_W}{Z_S + Z_W} \quad \text{bzw.} \quad T_{S,W} = \frac{2Z_S}{Z_S + Z_W}$$

Die Einzelimpedanzen können über Material- und geometrische Eigenschaften eingestellt werden. Bei der Annahme, dass der Stecker ein koaxiales System darstellt, kann von folgendem Leitungswellenwiderstand im Steckerbereich bei 64 MHz ausgegangen werden:

$$\left. |Z| \right|_{Stec\,ker} = \sqrt{\frac{\mu}{\underline{\varepsilon}_e}} \frac{1}{2\pi} \ln\left(\frac{D_{OC}}{D_{IC}}\right) = \frac{120\pi\Omega}{\sqrt{3} \cdot 2\pi} \cdot 0{,}606 = 20\Omega$$

Für gewendelte Bereiche ist der Wellenwiderstand aufgrund der Induktivitäts- und Kapazitätsbeläge wesentlich höher. Hier wurden messtechnisch $|Z|_{Wendel} \approx 150\ \Omega$ ermittelt. Für einen implantierbaren Generator (Herzschrittmacher, Cardioverter/Defibrillator, Neurostimulator etc.) muss aufgrund der Eingangsschaltung von einem sehr geringen Widerstand von $|Z|_{Generator} = 2 - 10\ \Omega$ ausgegangen werden.

Diese Leitungswellenwiderstände gelten für Leitungsbereiche, in denen die geometrischen und elektromagnetischen Eigenschaften konstant bleiben. Eine kontinuierliche Änderung des Leitungswellenwiderstands kann erzielt werden, wenn sich die geometrischen Eigenschaften über der Lauflänge verändern. Darüber hinaus können auch die elektromagnetischen Eigenschaften variieren. Leitungstheoretisch entsteht dabei ein Netzwerk aus parallel geschalteten Leitungswiderständen.

Ziel ist es über die geometrischen und elektrischen Eigenschaften die Leitungswellenwiderstände so einzustellen, dass sich ein kontinuierlicher Übergang von $|Z|_{Wendel}$ zu $|Z|_{Generator}$ einstellt. Das kann über geeignete Materialien, ein angepasstes Durchmesserverhältnis und durch den Übergang von koaxialen Wendeln zu koaxialen Rohren erreicht werden. Letztere weisen wesentlich geringere Induktivitätsbeläge auf. Mit $D_{OC} = 2{,}2$ mm und $D_{IC} = 0{,}1$ mm und einer relativen Permittivität $\varepsilon_r = 2$ von ergibt sich:

$$\left. |Z| \right|_{Stec\,ker} = \sqrt{\frac{\mu}{\underline{\varepsilon}_e}} \frac{1}{2\pi} \ln\left(\frac{D_{OC}}{D_{IC}}\right) = \frac{120\pi\Omega}{\sqrt{2} \cdot 2\pi} \cdot 3{,}1 = 131\Omega$$

Die Durchmesserverhältnisse können durch Variation der Induktivitätsbeläge der Wendel optimiert werden. Da die Wellenausbreitung zusätzlich durch die Leitfähigkeit oder Polarisationsverluste des Isolationsmaterials beeinflusst wird, kann die Amplitude der sich ausbreitenden Welle über der Leitungslänge gedämpft werden. Reflexionen an Grenzflächen durch unangepasste Leitungselemente wirken sich dadurch weniger stark aus.

Wenn der Übergang reflexionsarm und im Übergangsbereich zum Stecker dissipativ gestaltet werden soll, dann empfiehlt es sich den Leitungswellenwiderstand ähnlich hoch einzustellen. Unter der Berücksichtigung, dass sich die Koaxialgeometrie nicht ändert, geschieht dies vorzugsweise über die Materialeigenschaften der Füllung. Zusätzlich zu dem Leitungswellenwiderstand beeinflusst die Ausbreitungskonstante im Wellenleiter die Welle. Diese ist abhängig von den elektromagnetischen Parametern und kann folgendermaßen ausgedrückt werden:

$$\gamma = \omega\sqrt{\underline{\mu}_e\,\underline{\varepsilon}_e} = \omega\sqrt{\underline{\mu}_e\,(\varepsilon_0\Re(\varepsilon_r) + j(\varepsilon_0\Im(\varepsilon_r) + \frac{\sigma}{\omega\varepsilon_0}))} = \alpha + j\beta$$

γ = Ausbreitungskonstante
α = Dämpfungskonstante
β = Phasenkonstante

Imaginäranteile der effektiven Permittivität $\underline{\varepsilon}_e$ sowie die Leitfähigkeit gehen in die Dämpfungskonstante ein über eine erhöhte Phasenkonstante wird der Effekt von Polarisationsverlusten zusätzlich gesteigert, da sich diese anteilig pro Wellengang auswirkt.
Für Silikon ist die Ausbreitungskonstante:

$$\gamma_{Silikon} = j\omega\sqrt{\underline{\mu}_e\,\underline{\varepsilon}_e} = j\omega\sqrt{\mu_0\varepsilon_0\varepsilon_r} = 0 + 2{,}2j$$

Für Plexiglas oder einem mit erhöhter Leitfähigkeit dotiertem Silikon (σ = 0,1 Ω) sind die Ausbreitungskonstanten dissipativ und für den vorliegenden Anwendungsfall (f = 64 MHz) günstiger:

$$\gamma_{Plexiglas} = j\omega\sqrt{\underline{\mu}_e\,\underline{\varepsilon}_e} = 0{,}05 + 2{,}49j \ \text{ bzw. } \ \gamma_{Silikon,dotiert} = j\omega\sqrt{\underline{\mu}_e\,\underline{\varepsilon}_e} = 4{,}9 + 5{,}2j$$

Die Füllung besitzt vorzugsweise eine Impedanz, die an den Leitungswellenwiderstand angepasst ist. Die Impedanzanpassung ist vorzugsweise durch den Werkstoff (das Material) der Füllung bewirkt. Als Material der Füllung sind pyrolytische Werkstoffe, dotierte Kunststoffe mit para-, ferroelektrischen, dia-, ferro-, paramagnetischen Partikeln und Kunststoffe mit dielektrischen Verlusten bevorzugt.
Als Materialien der Füllung im Übergangsbereich zum Stecker bieten sich Flourkunststoffe (PTFE, Teflon) und thermoplastische Polykondensate an (PEEK, PEK) mit einer Dotierung aus folgenden Metallen an:

- Platin & Titan
- Refraktäre Materialien: Niob, Tantal, Palladium, Wolfram

Die Impedanz der Füllung ist vorzugsweise verlustbehaftet, so dass die Füllung dissipativ wirkt.
Die Dissipation wird über vorzugsweise über den Verlust (z.B. Leitfähigkeit) des Materials der Füllung eingestellt.
Die Leitfähigkeit der Materialien findet sich in folgender Tabelle:

| Pos. | Werkstoff | Leitfähigkeit in S/m | Quelle |
|------|-----------|----------------------|--------|
| 1. | Silber | $6{,}1 \cdot 10^6$ | [1] |
| 2. | Kupfer | $5{,}76 \cdot 10^6$ | [1] |
| 3. | Gold | $4{,}1 \cdot 10^6$ | [1] |
| 4. | Niob | $\cdot 10^6$ | [2] |
| 5. | Tantal | $0{,}69 \cdot 10^6$ | [1] |
| 6. | Palladium | $5{,}76 \cdot 10^6$ | [1] |
| 7. | Wolfram | $1{,}83 \cdot 10^6$ | [1] |
| 8. | Edelstahl | $0{,}115 \cdot 10^6$ | [1] |

(fortgesetzt)

| Pos. | Werkstoff | Leitfähigkeit in S/m | Quelle |
|------|-----------|----------------------|--------|
| 9. | Platin | $0,979 \cdot 10^6$ | [1] |
| 10. | Titan | $0,207 \cdot 10^6$ | [1] |
| [1]: White, Don: A Handbook on Electromagnetic Shielding Materials and Performance, Don White Consultants, Inc.; 2 edition (June 1980), ISBN 978-0932263087 [2]: Harry H. Binder: Lexikon der chemischen Elemente - das Periodensystem in Fakten, Zahlen und Daten. Hirzel, Stuttgart 1999, ISBN 3-7776-0736-3 | | | |

Bei geringerem Durchmesserverhältnis sollte vorzugsweise eine kleinere Permittivität des Isolationsstoffes der Füllung gewählt werden. Für ein dissipatives Verhältnis sollte das Material der Füllung ein (schlecht leitfähigen) Material sein, der Leitungswellenwiderstand soll sich hierbei so gering wie möglich ändern.

Die Dämpfungskonstante $\alpha$ hingegen sollte möglichst hoch sein:

$$\alpha_S = \frac{\omega}{\sqrt{2}} \sqrt{|\underline{\varepsilon}_e \, \underline{\mu}| - \mathrm{Re}(\underline{\varepsilon}_e \, \underline{\mu})}$$

wobei die Leitfähigkeit in die komplexwertige und effektive Permittivität $\varepsilon_e$ eingeht:

$$\underline{\varepsilon}_e = \varepsilon_e{}' - j\varepsilon_e{}''$$

$$\mathrm{mit}\ \varepsilon_e{}' = \varepsilon' \ \mathrm{und}\ \varepsilon_e{}'' = \varepsilon'' \frac{\sigma_s}{\omega}$$

Die Füllung ist vorzugsweise zwischen einem Innen- und einem Außenleiter angeordnet.

Vorzugsweise ist die Füllung so ausgebildet, dass sich deren Impedanz in Längsrichtung des Übergangsbereichs verändert, z.B. indem sich die Geometrie der Füllung Längsrichtung des Übergangsbereichs verändert.

Weiterhin ist es bevorzugt, wenn die Impedanz eine angepasste Wellenzahl eine hohe Reflexionsgüte und/ oder eine hohe Transmissionsgüte hat.

Vorzugsweise sind der oder die Funktionsleiter als Wendeln ausgeführt und bilden beispielsweise einen oder mehrere Innen- und Außenleiter einer Elektrodenleitung.

Alternativ können der oder die Funktionsleiter als parallel laufende Drähte ausgeführt sein und beispielsweise ebenfalls Innen- und Außenleiter einer Elektrodenleitung bilden. Ebenso können solche Innen- und Außenleiter als Kombination aus Wendel und parallel laufenden Drähten ausgeführt sein.

In allen Fällen kann die Windungsdichte der Wendeln konstant sein.

Um einen sich möglichst kontinuierlich verändernden Leitungswellenwiderstand zu bewirken, kann die Windungsdichte der Wendeln sich in Längsrichtung des Übergangsbereichs und ggf. auch darüber hinaus - verändern.

Zusätzlich zu Funktionsleitern können auch ggf. ebenfalls wendelförmige Zusatzleiter vorgesehen sein, die nicht oder nicht nur der Signalübertragung dienen und damit eine therapeutische Funktionshaben, sondern die die Einkopplung elektromagnetischer Wellen auf den oder die Funktionsleiter minimieren.

Die Erfindung soll nun anhand von Ausführungsbeispielen mit Bezug auf die Abbildungen näher erläutert werden. Die Abbildungen zeigen Folgendes:

Abb. 1    zeigt als implantierbare medizinische Geräte einen implantierbaren Herzstimulator 10 und eine daran angeschlossene implantierbare Elektrodenleitung 20.

Abb. 2    zeigt beispielhaft einen Temperaturverlauf an der Elektrodenspitze unter Einfluss von hochfrequenten Wechselfeldern, wie sie in einem Kernspintomografen (MRT) herrschen.

Abb. 3    zeigt ein proximales Ende einer Elektrodenleitung als implantierbares medizinisches Gerät, welches einen koaxialen Aufbau mit einem IS-1-Stecker aufweist und in einem Übergangsbereich zum Stecker eine Füllung mit angepassten elektromagnetischen Eigenschaften aufweist.

Abb. 4    zeigt ein proximales Ende einer Elektrodenleitung mit koaxialem Aufbau und IS-1-Stecker, welche eine Änderung des Durchmesserverhältnisses in einem Übergangsbereich zum Stecker aufweist.

Abb. 5    zeigt ein proximales Ende einer Elektrodenleitung mit koaxialem Aufbau und IS-1-Stecker, welche eine Änderung der Füllungseigenschaften in Längsrichtung des Übergangsbereiches zum Stecker aufweist.

Abb. 6    zeigt ein proximales Ende einer Elektrodenleitung mit IS-4/DF-4-Stecker, welche außer einer Änderung der Füllungseigenschaften in Längsrichtung des Übergangsbereiches zum Stecker auch parallel geführte Drähte und eine Innenwendel aufweist.

Abb. 7    zeigt ein proximales Ende einer Elektrodenleitung mit IS-4/DF-4-Stecker, welche eine Änderung der Drahtführung aufweist. Die Drähte können beispielsweise verdrillt, mit variabler Steigung oder mit nicht therapeutisch genutzten Drähten kombiniert werden.

Abb. 8    zeigt ein proximales Ende einer Elektrodenleitung mit IS-4/DF-4-Stecker, welche eine Änderung der Füllungseigenschaften und der Drahtführung aufweist.

Abb. 9    zeigt ein Ersatzschaltbild für ein Netzwerk aus Leitungswiderständen,

Abb. 10    zeigt die Wellenausbreitung an Grenzflächen ohne dämpfenden Stecker, dargestellt über die Amplitude der elektrischen Feldkoordinate die Eingangsamplitude ist normiert auf 1 V/m,

Abb. 11    zeigt Wellenausbreitung an Grenzflächen mit dämpfendem Stecker, dargestellt über die Amplitude der elektrischen Feldkoordinate die Eingangaamplitude ist normiert auf 1 V/m,

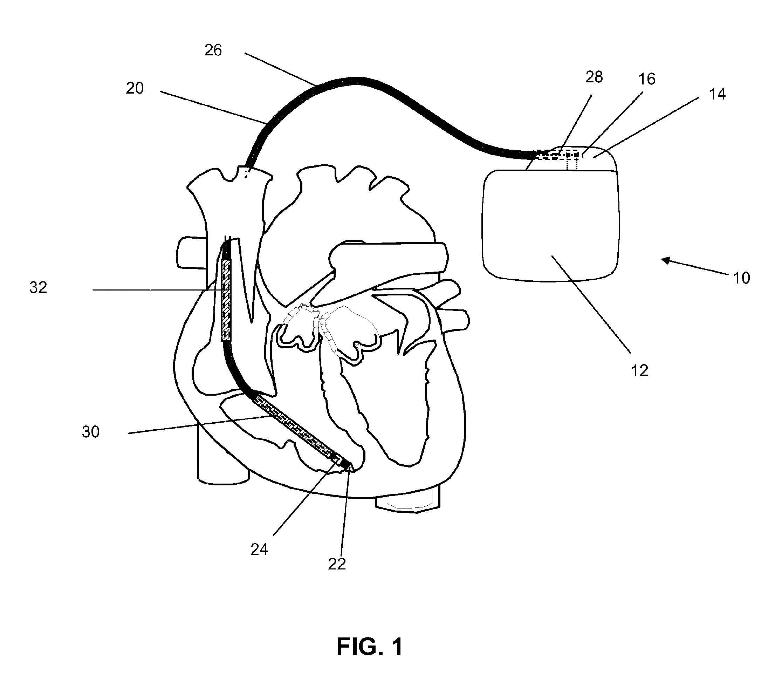

In Abbildung 1 ist ein implantierbares medizinisches Gerät in Form einer Elektrodenleitung 20 dargestellt, die einen langgestreckten Leiter aufweist und an weiteres implantierbares medizinisches Gerät, nämlich einen implantierbaren Herzstimulator 10, angeschlossen ist.

Der implantierbare Herzstimulator 10 kann ein Herzschrittmacher oder ein Kardioverter/Defibrillator (ICD) sein. In dem in Abbildung 1 dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ein implantierbares medizinisches Gerät mit einem langgestrecktem, elektrischem Funktionsleiter dar. An einem distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Abb. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Kontakt eines Steckers 28 am proximalen Ende der Elektrodenleitung 20 elektrisch verbunden. Der Stecker 28 besitzt elektrische Kontakte, die mit den elektrischen Kontakten 16 der Kontaktbuchse im Anschlussgehäuse 14 des implantierbaren Herzstimulators korrespondieren. Stecker für Elektrodenleitungen standardisiert und werden entsprechend dem jeweiligen Standard als IS-1-Secker oder als IS-4 bzw. DS-4 Stecker bezeichnet. Einschlägige Standards sind DIN 50077 und ISO 27186.

Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

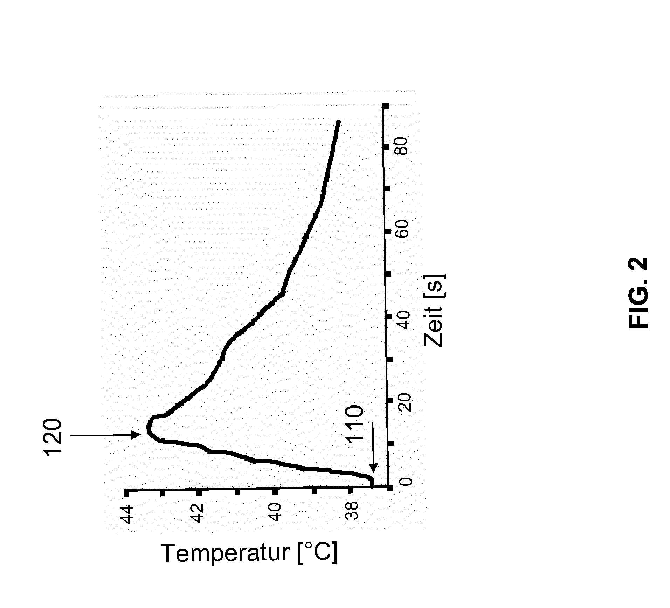

In Abbildung 2 ist ein typischer Temperaturverlauf 100 einer konventionellen Schrittmacher/ICD-Elektrode in einem Kernspintomografen (Magnetresonanztomografen, MRT) dargestellt. Mit dem Einschalten des hochfrequenten Wechselfeldes im Tomografen zum Zeitpunkt 110 steigt die Temperatur rasch an, wobei die Steilheit des Anstieges und die maximal erreichbare Temperatur stark von der Elektrodenlage bezogen auf die hochfrequenten Wechselfelder des MRT abhängig ist. Wird das hochfrequente Wechselfeld abgeschaltet (zum Zeitpunkt 120), dann kühlt sich die Elektrodenspitze aufgrund ihrer vergleichsweise geringen Wärmekapazität verhältnismäßig schnell wieder ab.

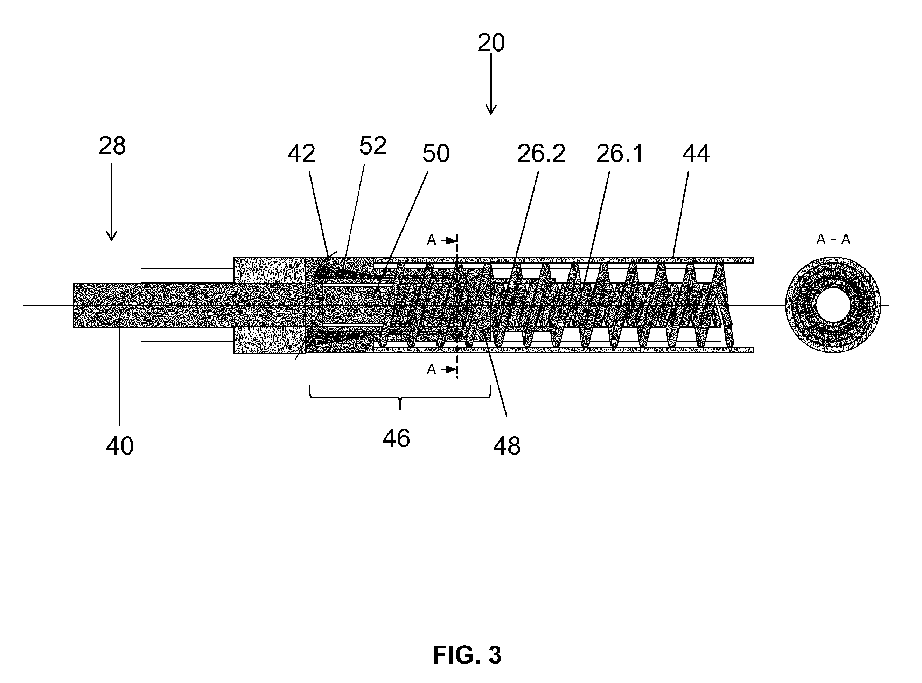

Abbildungen 3 bis 8 zeigen verschiedene Ausführungsvarianten eines jeweiligen proximalen Endes einer Elektrodenleitung mit IS-1 bzw. IS-4-Stecker.

Abbildung 3 zeigt das proximale Ende einer Elektrodenleitung 20 mit einem IS-1-Stecker gemäß DIN 50077. Der Stecker 28 besitzt einen ersten stiftförmigen Kontakt 40 und einen zweiten ringförmigen Kontakt 42, die voneinander isoliert sind. Der stiftförmige Kontakt 40 ist elektrisch mit einem gewendelten Innenleiter 26.1 verbunden. Der ringförmige Kontakt 42 ist mit einem ebenfalls gewendelten Außenleiter 26.2 verbunden. Innenleiter und Außenleiter sind beides Funktionsleiter der Elektrodenleitung 20 und sind von einer isolierenden Hülle 44 umgeben. Ein distaler, mit dem Innenleiter 26.1 und dem Außenleiter 26.2 in Verbindung stehender Abschnitt des Steckers 28 ist koaxial aufgebaut und bildet einen Übergangsbereich 46 vom Stecker 28 zur übrigen Elektrodenleitung 20.

In dem Übergangsbereich 46 umgibt der ringförmige Kontakt 42 einschließlich eines Anschlusses Abschnitts 48 den stiftförmigen Kontakt 40 und dessen Anschluss Abschnitt 50 zum Anschluss des Innenleiters 26.1. Zwischen dem ringförmigen Kontakt 42 in seinem äußeren Anschluss Abschnitt 48 sowie in dem hier von eingeschlossenen stiftförmigen Kontakt 40 mit seinem inneren Anschluss Abschnitt 50 ist eine Füllung 52 vorgesehen, deren elektromagnetische und geometrische Eigenschaften so eingestellt sind, dass sich der Wellenwiderstand der Elektrodenleitung 20 im Übergangsbereich 46 kontinuierlich ändert, so dass es zu einer möglichst kontinuierlichen Anpassung des Leitungswellenwiderstandes der distal gelegenen Abschnitte der Elektrodenleitung 20 und eines bei angeschlossenem Stecker 28 proximal herrschenden Leitungswellenwiderstand kommt.

Wie Abbildung 3 zu entnehmen ist, wird diese Anpassung des Wellenwiderstandes unter anderem durch eine Geometrie der Füllung 52 bewirkt, bei der das Volumen der Füllung im Bereich des ringförmigen Kontaktes 42 in proximaler Richtung zunimmt. Die Füllung 52 ist isolierend und besteht aus einem Werkstoff beziehungsweise aus einem Material, das an anderer Stelle genauer beschrieben ist und das eine geeignete Impedanz aufweist, die verlustbehaftet ist, so dass die Füllung 52 dissipativ wirkt.

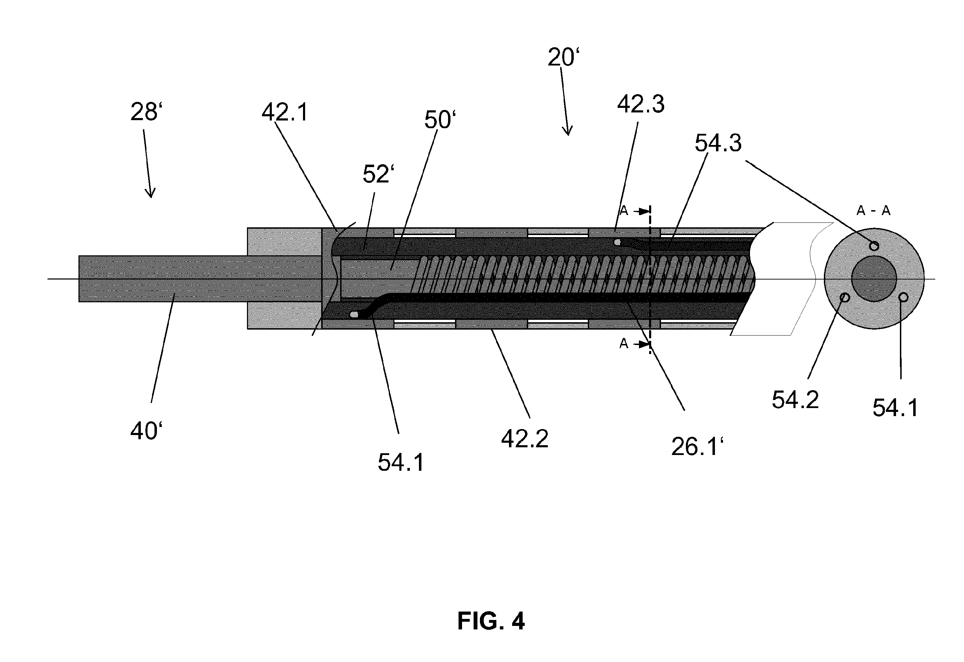

Abbildung 4 zeigt einen proximalen Endabschnitt einer Elektrodenleitung 20' mit einem Stecker 28', der als IS-4-Stecker gemäß ISO 27186 herausgebildet ist. Der Stecker besitzt einen stiftförmigen Kontakt 40' und insgesamt drei ringförmige Kontakte 42.1, 42.2 und 42.3. Der stiftförmige Kontakt 28' ist an seinem distalen Ende mit einem gewendelten Innenleiter 26.1' verbunden. Die ringförmigen Kontakte 42.1, 42.2 und 42.3 sind jeweils mit einem in Längsrichtung der Elektrodenleitung 20 verlaufenden Draht 54.1, 54.2 und 54.3 verbunden. Die Drähte 54.1, 54.2 und 54.3 stellen Außenleiter dar und sind Funktionsleiter. Zwischen den ringförmigen Kontakten 42.1, 42.2 und 42.3 und einem distalen Anschlussabschnitt 50' des stiftförmigen Kontaktes 28 sowie dem daran angeschlossenen, gewendelten Innenleiter 26.1' ist eine Füllung 52 aus einem isolierenden Material mit angepassten, elektromagnetischen Eigenschaften vorgesehen. Geeignete Materialien und Eigenschaften sind an anderer Stelle dieses Textes näher beschrieben.

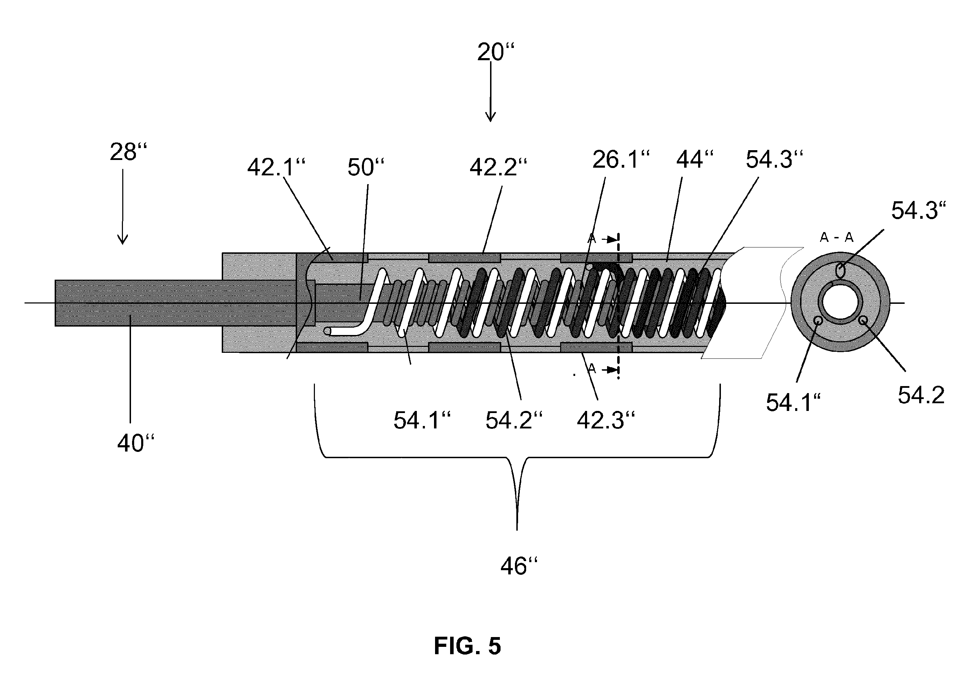

Abbildung 5 zeigt eine Variante einer Elektrodenleitung 20" mit einem IS-4-Stecker 28" ähnlich der Anordnung in Abbildung 4. Im Unterschied zu der Ausführungsvariante gemäß Abbildung 4 sind die außenliegenden Funktionsleiter 54.1, 54.2 und 54.3 jeweils gewendelt. Die Windungsdichte der die Außenleiter 54.1', 54.2' und 54.3' bildenden Wendeln kann dabei variieren, so dass auf diese Weise eine Variation des Leitungswellenwiderstandes in Längsrichtung des Übergangsbereichs 42" bewirkt wird. Bei den Abbildungen 5 dargestellten Ausführungsvariante muss nicht unbedingt eine Füllung in dem Zwischenraum zwischen den ringförmigen Kontakten 54.1", 54.2" und 54.3" einerseits und dem inneren Anschluss Abschnitt 50" des stiftförmigen Steckkontakts 40" beziehungsweise den daran angeschlossenen, gewendelten Innenleitern 26.1' vorgesehen sein.

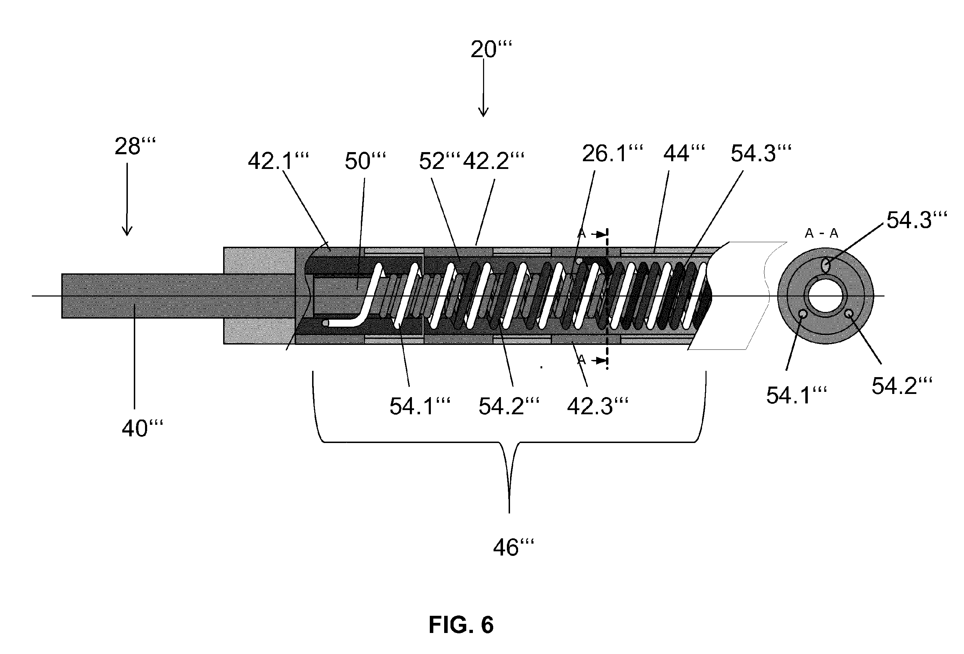

Bei der in Abbildung 6 dargestellten Ausführungsvariante ist solch eine Füllung 52''' vorgesehen, deren elektromagnetische Eigenschaften in Längsrichtung des Übergangsbereichs 42''' variieren, so dass sich eine Variation des Leitungswellenwiderstandes und damit eine möglichst gleichmäßige Anpassung des Leitungswellenwiderstandes der distal gelegenen Elektronenleitung 20''' und des proximalen Steckers 28''' herrschenden Leitungswellenwiderstandes ergibt. Bis auf die Füllung 42''' entspricht die Ausführungsvariante gemäß Abbildung 6 der Ausführungsvariante gemäß Abbildung 5.

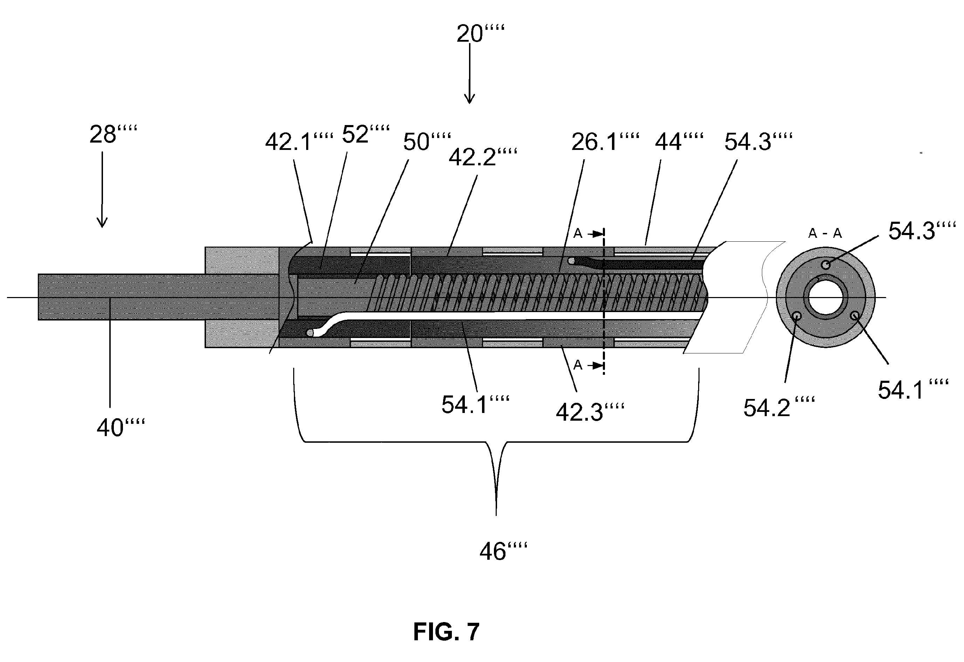

Ähnliches gilt für die Ausführungsvariante gemäß Abbildung 7, die bis auf eine Füllung 52'''' der Ausführungsvariante in Abbildung 4 entspricht. Die Füllung 52'''' besitzt in Längsrichtung des Übergangsabschnittes 46'''' variierende, elektromagnetische Eigenschaften, so dass auch der Leitungswellenwiderstand in diesem Übergangsbereich variiert und sich ein möglichst gleichmäßiger Übergang zwischen dem Leitungswellenwiderstand des distal des Übergangsabschnittes 46'''' gelegenen Abschnitts der Elektrodenleitung 20 und des proximal des Übergangsbereichs 46'''' herrschenden Leitungswellenwiderstands ergibt, wenn der Stecker 28'''' in ein weiteres Gerät eingesteckt ist.

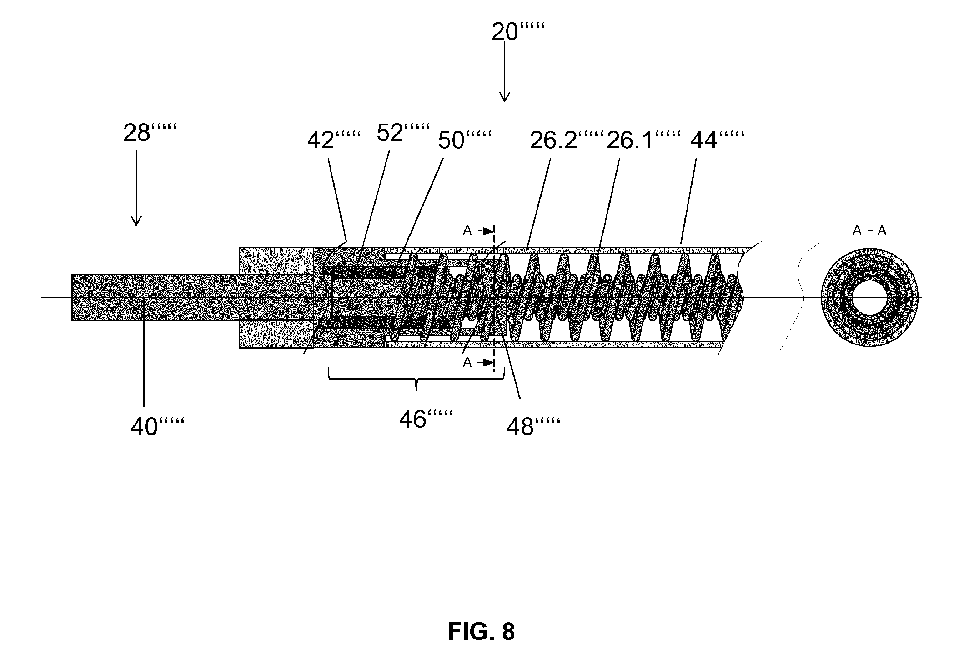

Abbildung 8 zeigt eine Ausführungsvariante, die der Ausführungsvariante nach Abbildung 3 ähnlich ist. Die beiden Ausführungsvarianten unterscheiden sich durch die Gestaltung der Füllung 52. Während bei der Ausführungsvariante gemäß Abbildung 3 die Geometrie der Füllung in Längsrichtung des Übergangsbereichs 46 variiert, variieren bei der Ausführungsvariante aus Abbildung 8 die elektromagnetischen Eigenschaften der Füllung in Längsrichtung des Übergangsbereichs 46''''.

Wie eingangs ausgeführt sollte für die isolierende Füllung 52 im Übergangsbereich zum Stecker jeweils ein Material ausgewählt werden, dass den Leitungswellenwiderstand im koaxialen Bereich sinnvoll verändert. Aufgrund solch einer Änderung ändert sich auch der jeweilige Reflexions- und Transmissionsfaktor einen Bereich zwischen $R_{S,W}$ [0;1] und $T_{S,W}$[-1,1].

Der Faktor ist dabei abhängig von der Differenz der Leitungswellenwiderstände zwischen Stecker $Z_S$ und Wendelbereich $Z_W$.

In den Ausführungsbeispielen ist der jeweilige Übergangsbereich 46 vorzugsweise jeweils reflexionsarm und im Steckerbereich dissipativ ausgebildet. Hierzu ist der dort herrschende Leitungsellenwiderstand ähnlich hoch eingestellt, wie in benachbarten Leitungsabschnitten. Unter der Berücksichtigung, dass sich die Koaxialgeometrie ändert, muss dies über die Materialeigenschaften geschehen.

Geeignete Materialien für die jeweilige Füllung 52 sind Flourkunststoffe (PTFE, Teflon) und thermoplastische Polykondensate an (PEEK, PEKK) mit einer Dotierung aus folgenden Metalle:

- Platin & Titan
- Refraktäre Materialien: Niob, Tantal, Palladium, Wolfram

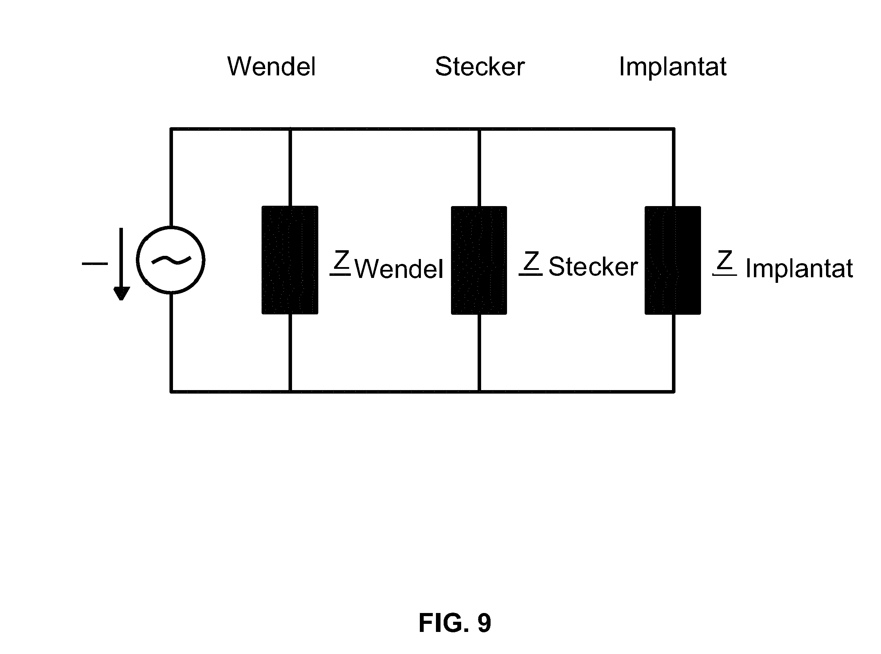

Zur Verdeutlichung des Wirkprinzips zeigt die Abbildung 9 ein Netzwerk aus Leitungswellenwiderständen, wie es für die Berechnungen verwendet wurde.

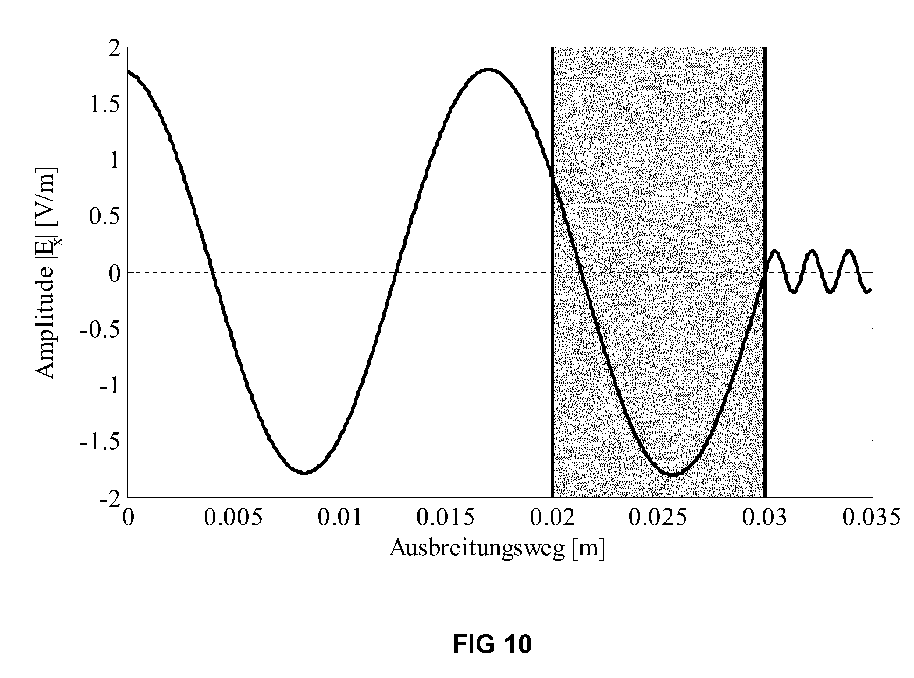

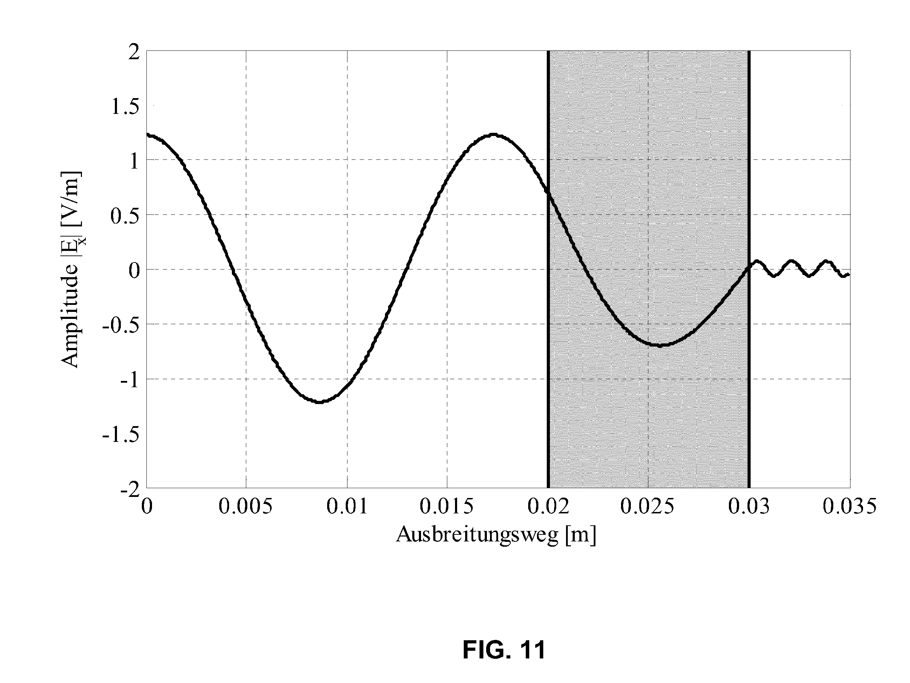

Die Abb. 10 zeigt die Wellenausbreitung an Grenzflächen ohne dämpfenden Stecker, dargestellt über die Amplitude der elektrischen Feldkoordinate die Eingangsamplitude ist normiert auf 1 V/m und die Abb. 11 zeigt die Wellenausbreitung an Grenzflächen mit dämpfendem Stecker, dargestellt über die Amplitude der elektrischen Feldkoordinate die Eingangaamplitude ist normiert auf 1 V/m. Die Abb. 10 zeigt das Verhalten bei starker Reflexion am Eingang des Implantats ohne Dämpfung über der Steckerlänge. Die resultierende Feldstärke im Wendelbereich ist fast doppelt so groß wie die Eingangsfeldstärke. In Abb. 11 ist der Steckerbereich dissipativ gestaltet. Die resultierende Feldstärke im Wendelbereich ist wesentlich geringer. Der in den Abb. 10 und Abb. 11 grau dargestellte Bereich kennzeichnet den Steckerbereich, der weiße Bereich links davon jeweils den Wendelbereich und der Bereich recht vom grauen Bereich jeweils den Eingang vom IPG. Der Nullpunkt auf der Abszisse bezeichnet den Anfang des Steckers und die elektromagnetische Welle läuft von links nach rechts.

**Patentansprüche**

**1.** Temporär in einen Körper einführbares oder dauerhaft in einen Körper implantierbares Gerät mit wenigstens einem langgestreckten elektrischen Funktionsleiter für die Übertragung von Therapiesignalen oder Diagnosesignalen oder beidem, sowie mit einem mit dem Funktionsleiter verbundenen und an einem proximalen Ende des Funktionsleiters angeordneten Stecker zum Anschließen des Gerätes an ein weiteres Gerät, wobei zwischen dem Funktionsleiter und wenigstens einem weiteren Leiter ein Leitungswellenwiderstand herrscht, **dadurch gekennzeichnet, dass** ein Übergangsbereich von dem Funktionsleiter zu dem Stecker derart ausgebildet ist, dass ein Leitungswellenwiderstand zwischen dem Funktionsleiter und dem weiteren Leiter in dem Übergangsbereich so bemessen ist, dass er zwischen dem entsprechenden Leitungswellenwiderstand des Gerätes in einem distal des Übergangsbereichs gelegenen Leitungsabschnitt und dem Leitungswellenwiderstand liegt, der bei an ein weiteres Gerät angeschlossenem Stecker proximal des Übergangsbereichs herrscht.

2. Implantierbares medizinisches Gerät nach Anspruch 1, **dadurch gekennzeichnet, dass** das Gerät eine Elektrodenleitung zum Anschluss an ein implantierbares medizinisches Gerät ist, wobei die Elektrodenleitung an ihrem proximalen Ende den Stecker aufweist und an ihrem distalen Ende wenigstens einen Elektrodenpol, der über den wenigstens einen Funktionsleiter mit dem Stecker elektrisch verbunden ist.

3. Implantierbares medizinisches Gerät nach Anspruch 1 oder 2, **dadurch gekennzeichnet, dass** im Übergangsbereich des Gerätes eine Füllung angeordnet ist, die den Leitungswellenwiderstand beeinflusst und deren Eigenschaften und Form so gewählt sind, dass sich der gewünschte Leitungswellenwiderstand im Übergangsbereich ergibt.

4. Implantierbares medizinisches Gerät nach Anspruch 3, **dadurch gekennzeichnet, dass** die Füllung vorzugsweise Impedanz besitzt, die an den Leitungswellenwiderstand angepasst ist.

5. Implantierbares medizinisches Gerät nach Anspruch 3 oder 4, **dadurch gekennzeichnet, dass** die Füllung ein Material enthält, das wenigstens in einem Arbeitsfrequenzbereich von Kernspintomografen dissipativ wirkt.

6. Implantierbares medizinisches Gerät nach Anspruch 4 oder 5, **dadurch gekennzeichnet, dass** das Material der Füllung pyrolytische Werkstoffe, dotierte Kunststoffe mit para-, ferroelektrischen, dia-, ferro-, paramagnetischen Partikeln oder Kunststoffe mit dielektrischen Verlusten oder Flourkunststoffe und/oder thermoplastische Polykondensate mit einer Dotierung aus einem oder mehreren der folgenden Metallen enthält:

   Platin, Titan und refraktäre Metalle wie: Niob, Tantal, Palladium und Wolfram.

7. Implantierbares medizinisches Gerät nach einem der Ansprüche 1 bis 6, **dadurch gekennzeichnet, dass** der Übergangsbereich koaxial aufgebaut ist.

8. Implantierbares medizinisches Gerät nach einem der Ansprüche 3 bis 7, **dadurch gekennzeichnet, dass** die Füllung zwischen einem Innen- und einem Außenleiter angeordnet ist.

9. Implantierbares medizinisches Gerät nach einem der Ansprüche 3 bis 8, **dadurch gekennzeichnet, dass** die Füllung so ausgebildet ist, dass sich deren Impedanz in Längsrichtung des Übergangsbereichs verändert.

10. Implantierbares medizinisches Gerät nach einem der Ansprüche 1 bis 9, **dadurch gekennzeichnet, dass** ein oder mehrere Funktionsleiter als Wendeln ausgeführt sind.

11. Implantierbares medizinisches Gerät nach einem der Ansprüche 1 bis 10, **dadurch gekennzeichnet, dass** ein oder mehrere Funktionsleiter als parallel laufende Drähte ausgeführt sind.

12. Implantierbares medizinisches Gerät nach Anspruch 10, **dadurch gekennzeichnet, dass** die Windungsdichte einer oder mehrerer Wendeln konstant ist.

13. Implantierbares medizinisches Gerät nach Anspruch 10 oder 12, **dadurch gekennzeichnet, dass** die Windungsdichte einer oder mehrerer Wendeln sich in Längsrichtung des Übergangsbereichs verändert.

14. Implantierbares medizinisches Gerät nach einem der Ansprüche 1 bis 13, **dadurch gekennzeichnet, dass** zusätzlich zu dem wenigstens einen Funktionsleiter wenigstens ein Zusatzleiter vorgesehen ist, der nicht oder nicht nur der Signalübertragung dient, sondern die die Einkopplung elektromagnetischer Wellen auf den wenigstens einen Funktionsleiter minimiert.

**15.** Implantierbares medizinisches Gerät nach Anspruch 14, **dadurch gekennzeichnet, dass** der wenigstens eine Zusatzleiter gewendelt ist.

**FIG. 1**

EP 2 505 229 A2

FIG. 2

20

28

44

26.1

26.2

50

52

42

A - A

48

46

40

**FIG. 3**

FIG. 4

FIG. 5

**FIG. 6**

EP 2 505 229 A2

FIG. 7

FIG. 8

Wendel     Stecker     Implantat

$\underline{Z}$Wendel    $\underline{Z}$ Stecker    $\underline{Z}$ Implantat

**FIG. 9**

EP 2 505 229 A2

FIG 10

**FIG. 11**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **WHITE, DON.** A Handbook on Electromagnetic Shielding Materials and Performance. Don White Consultants, Inc, Juni 1980

- **HARRY H. BINDER ; FAKTEN, ZAHLEN ; DATEN. HIRZEL.** Lexikon der chemischen Elemente - das Periodensystem in. 1999